# EUROPEAN PATENT APPLICATION

(11) **EP 3 874 954 A1**
(43) Date of publication of application: **08.09.2021**
(21) Application number: 19880217.5
(22) Date of filing: 28.10.2019
(51) Int. Cl.: A01N 63/30, A01N 25/08, A01N 25/10

(54) **SOLID COMPOSITION FOR PLANT CARE CONTAINING FUNGAL SCLEROTIUM AND USE THEREOF**

(30) Priority: 01.11.2018 KR 20180132686
(71) Applicant: Moghu Research Center Ltd., Yuseong-gu, Daejeon 34115 (KR)
(72) Inventor: KIM, Dal-Soo, Seongnam-si, Gyeonggi-do 13565 (KR)
(74) Representative: Nony
(86) International application number: PCT/KR2019/014258
(87) International publication number: WO 2020/091335

(57) **Abstract**

The present invention is regarding sclerotium composition which is able to weed out target plants, including the Bur Cucumber which is designated as an ecologically damaging plant and suppress soil-borne diseases like Sclerotinia dollar spot disease, more specifically by the suitable use of sclerotium of sclerotium-forming fungi and composition including nutrients for sclerotium-forming fungi and absorbent materials, Bur Cucumber and Sclerotinia dollar spot disease can effectively be controlled, and it is able to make significant gains in solving problems with biological products containing microorganisms in stages of production, storage, distribution, and usage process.

## Description

### [TECHNICAL FIELD]

The present invention relates to a solid composition containing sclerotium used for plant management and its uses, among various plant management methods it specifically relates to the biological method in controlling weeds and soil-borne diseases. The present invention relates to a solid composition containing fungal sclerotium which is able to effectively inhibit the growth of designated ecologically harmful plants such as Bur Cucumber (*Sicyos angulatus*) and other target plants to be managed, and also effectively suppress the occurrence of soil-borne diseases like the Sclerotinia dollar spot (*Sclerotinia homoeocarpa*). In addition, the present invention relates to a solid composition containing fungal sclerotium which forms fungus, nutrients necessary for the growth of fungus strain mentioned above, and absorbent materials that can accommodate both the fungal sclerotium and the nutrients and the application of the solid composition itself.

### [TECHICAL BACKGROUND]

There are about 3,611 species of plants in Korea, 18 (6.0%) of them are vines, 135 species are herbaceous plants, and 83 species are woody plants.

However, in the distribution chart of prominent ecologically harmful plants to be managed, which include the plants mentioned above, released by the Korean Forest Service, kudzu (*Pueraria lobata*), Bur Cucumber (*Sicyos angulatus,* EPPO code: SIYAN), and Japanese hop (*Humulus japonicas*) each respectively take up 1.5%, 1% and 1% of the total land, and their share is gradually increasing, so controlling them is reported as being urgent (Kang Byung-hwa, 2014).

Among them, Bur Cucumber is best known as a foreign invasive plant that is ecologically disturbing, and ecologically it mainly occurs along riversides and is widely distributed on roadsides, vacant land, and farmland.

The seed of Bur Cucumber is dormant because its hard seed coat makes it have low water absorbency so the seed hardly germinates on the year it was formed and survives in the soil for 3 to 7 years, and it is an annual plant that can grow very quickly, sometimes growing more than 30 cm per day. Also, it can grow to a total length of more than 20 m and up to 80,000 seeds can be produced by one individual plant, and it can be transported widely by water, animals, people, etc. In recent years, the plant has been reported to spread rapidly around the Han river system and the Nakdong river system and it is considered an important target plant to be managed in each region's representative water system. Because of this, each municipality is actively promoting projects to control the Bur Cucumber, but the reality is that the project's effectiveness is limited mainly due to the lack of effective methods.

To date, known Bur Cucumber control methods include physical methods such as hand weeding, plowing, machine cutting, etc., chemical methods using herbicidal substances, biological methods using plant pathogens with high host specificity, etc., but mostly physical methods are used in practice which have problems such as labor costs, worker risk exposure, limitation of work range, and time constraints, etc.

Among them, the biological method of using microorganisms consists of evaluating the pathogenicity to the Bur Cucumber and selecting the plant pathogenic microbe that can selectively cause disease for the Bur Cucumber, the method known so far has a very demanding process of production, storage, distribution, etc. for the plant pathogenic microorganisms and its effects vary heavily depending on the environment, so commercially successful cases were very rare by now. This is because the plant pathogenic microbes are generally affected greatly by environmental factors such as temperature, humidity, and direct sunlight when commercialized with living microorganisms. Especially when applied in natural conditions, the stability and effectiveness of the original product can only be expected for a very short period of time, and the expectations for long-term efficacy were low. In addition, when high temperature or pressure is applied during the production process, distribution, or storage period of microbial formulation products, because the biological activity of the microbial can be reduced or inactivated. Inappropriate temperature, humidity or direct exposure to sunlight while in storage period or in the distribution process is undesirable and needs to be avoided. Because of these limitations, most biological formulations had a very short shelf life and particular management such as refrigerated storage was necessary.

However, for some of the fungi, unlike other fungi, there are fungi that form sclerotia at a certain growth stage of their life cycle (hereinafter referred to as 'sclerotium-forming fungi'), the fungal sclerotium is generally defined as mycelial mass hardened compact during its growth. This fungal sclerotium is formed for survival in unfavorable conditions and when the growing environment changes to an ecologically favorable state, it has the property of germinating and growing into mycelia. According to Smith et al. (2015), after an analysis of the phylogenetic linkage and nutritional characterization of fungi that form sclerotium, sclerotium-forming fungi was found to be ecologically diverse and taxonomically interspersed extensively in about 20 orders and 85 genera. Among these sclerotium-forming fungi, the genera Sclerotinia, Sclerotium, Rhizoctonia and Botrytis are representative plant pathogens and have pathogenicity to respective host plants. However, so far, the sclerotium of sclerotium-forming fungi has not been reported in having utility for plant management by itself, only the composition of the mycelial culture in the laboratory or the suspension of the mycelium in experiments have been reported as having an inhibiting effect of the target plant. Namely, *Sclerotinia trifoliorum* BWC98-105 strain which taxonomically belongs to the Ascomycetes is reported as having relatively high selective pathogenicity to Leguminosae such as clover (Korea Patent No. 100496011, *Sclerotinia trifoliorum* BWC98-105 with herbicidal activity against clover and the method of producing mycelial suspension for clover herbicide using the same strain), in the pathogenicity evaluation, there was no pathogenicity to Monocotyledonous plants such as rice, barley, corn, wheat and grass, but Dicotyledonous legumes such as soybean, peanut, pea and cowpea had weak pathogenic properties, and there was no pathogenic properties to weeds in the poaceae family like cockspur grass, long-awned barnyard grass, and rice cockspur grass, but there were some pathogenic properties to Japanese cutgrass and weedy rice. *Cyperus difformis*, *Cyperus serotinus, Fimbristylis littoralis, Schoenoplectus juncoides,* and *Eleocharis kuroguwai* in Cyperaceae, *Monochoria plantaginea* and *Monochoria vaginalis* of Pontederiaceae, *Aeschynomene indica* and *Trifolium repens* of Fabaceae, *Sagittaria trifolia* and *Sagittaria sagittifolia* of Alismataceae, *Commelina communis* of Commelinaceae, *Polygonum hydropiper* of Polygonaceae, and *Typha shuttleworthii* of Typhaceae were pathogenic, while *Ludwigia prostrata* of Onagraceae and *Bidens tripartiata* of Asteraceae were recorded as not being pathogenic.

Additionally, the sclerotium-forming fungi, *Sclerotium delphinii* BWC04-107 which taxonomically belongs to the Basidiomycetes, in experiments using mycelium suspension of the same strain, it was reported as having relatively high selective pathogenicity to Conyza species (Asteraceae) that are resistant to the herbicide paraquat (Korean Patent No. 100769363, *Sclerotium delphinii* BWC04-107 strain and mycelial suspension prepared using the same strain, used for control of paraquat-resistant Conyza species), in pathogenicity evaluation results potatoes, kidney beans, sweet potatoes, oats, proso, mung bean, cowpea, wheat, rice, barley, sorghum, corn, pea, jack bean, broad bean, foxtail millet, soybean, adzuki beans, tobacco, Korean perilla, peanut, adlay, sesame, eggplant, chili pepper, carrot, strawberry, garlic, radish, Chinese cabbage, Chinese chives, lettuce, watermelon, and cabbage were not pathogenic, but cucumbers were pathogenic, among the weeds *Potamogeton distinctus* of Potamogetonaceae, *Solanum nigrum* of Solanaceae, *Spirodela polyrhiza* of Lemnaceae, *Juncus effusus* of Juncaceae, *Oxalis corniculata* of Oxalidaceae, *Ambrosia trifida, Bidens frondosa, Taraxacum officinale, Taraxacum ohwianum,* and *Taraxacum platycarpum* of Asteraceae, *Eleocharis kuroguwai, Schoenoplectus juncoides* of Cyperaceae, *Equisetum arvense* of Equisetaceae, *Arabidopsis thaliana* of Brassicaceae, *Portulaca oleracea* of Portulacaceae, *Sagittaria sagittifoila* of Alismataceae, *Commelina communis* of Commelinaceae, *Acalypha australis* of Euphorbiaceae, *Persicaria hydropiper, Polygonum aviculare, Rumex acetosa,* and *Rumex acetosella* of Polygonaceae, *Cuscuta japonica, Cuscuta pentagona, Cuscuta australis,* and *Cuscuta chinensis* of Convolvulaceae, *Chenopodium album* of Chenopodiaceae, *Eichhornia crassipes, Monochoria korsakowii,* and *Monochoria vaginalis* of Pontederiaceae, *Alopecurus aequalis, Avena fatua, Digitaria ciliaris,* and *Echinochloa* species of Poaceae were not pathogenic, but *Conyza canadensis, Conyza parva, Conyza bonariensis, Erigeron annuus, Artemisia capillaris, Artemisia dubia, Artemisia princeps, Artemisia selengensis, Gnaphalium affine, Gnaphalium uliginosum* of Asteraceae, and *Aeschynomene indica* and *Trifolium repens* of Fabaceae were recorded as being patheogenic.

In the case of countries abroad using sclerotium-forming fungi, Sclerotium rolfsii SC64 strain has proven its potential as a bio-herbicide for broadleaf weed and has been tested for pathogenicity in various weeds in plot scale experiments, but this experiment is characterized as using mycelial preparations rather than sclerotium (Tang et al. 2011, Field evaluation of Sclerotium rolfsii, a biological control agent for broadleaf weeds in dry direct-seeded rice. Crop Protection 30, 1315-1320.).

US Patent No. 5994267, 8557735 reported that the Sclerotinia minor IMI344141 strain has a weeding effect that effectively controls broad leaf weeds, including dandelion varieties of the Asteraceae family, but the result is from activated mycelium in form of a formulation that is prepared in the form of particles of about 1.7 mm in diameter by culturing mycelium in a medium containing grain, crushing and mixing additional medium ingredients, being applied over a grass field in concentrations of 20∼60g /m2 but not using the sclerotium itself. That is, the above U.S. patent composition consists of crushed barley, millet, rice or wheat mixed with sodium alginate or kaolin (kaolin clay) to form particles with sticking agent being applied to the surface of the particles, but the microbial survival period is less than 11 days, and the storage period is only about 14 days, limiting industrial use.

Establishing competitive condition between plant pathogens and biocontrol microorganisms for the same habitat space and nutrients is an alternative methods of biological methods for plant disease management. Major diseases that occur on golf course grass in Korea include large patch; pathogen *Rhizoctonia solani,* dollar spot; pathogen *Sclerotinia homoeocarpa,* etc., and it is difficult to replace the golf course grass for management of these diseases so these soil-borne diseases are generally managed with chemical pesticides, therefore there is a large concern about chemical pollution. The pathogen *Sclerotinia homoeocarpa* that causes dollar spot in grass and both the *Sclerotinia trifoliorum* 98-105 (*Sclerotinia trifoliorum* BWC98-105) strain used in the composition of the present invention belong to the same genus taxonomically, and the BWC98-105 strain is expected to do no damage to the grass when using it from the description of the Korean Patent No. 100496011 that the BWC98-105 strain has no pathogenicity to the grass. Therefore, when applying the composition of the present invention comprising the sclerotium of the BWC98-105 strain before the occurrence of the dollar spot disease, the strain settles on the soil where the grass grows and surface space, when the dollar spot disease occurs later, both the BWC98-105 strain and the dollar spot disease pathogen become competitive for the same habitat and nutrients which limits the occurrence of the dollar spot disease.

The present invention has been developed to provide a foundation for the suppression of soil-born diseases as well as weed management by using the sclerotium itself, in particular, by using the sclerotium itself in a dormant state that is not activated due to its hardened shell in contrast to the previous use with mycelial preparation, there is a characteristic that the production, storage and distribution phase, and field application can proceed conveniently without much concern for the biological deactivation.

### [DETAILED DESCRIPTION OF THE INVENTION]

### Technical Problem

In order to solve the above problems, the present invention uses sclerotia formed at a certain growth stage in the life cycle of the fungus, but also include the nutrients.

In addition, the present invention, by including the absorbent material, the efficacy is stably and effectively expressed when the sclerotium is activated and the present invention also seeks to improve the overall management cost and convenience of the production, storage, distribution, and application stages.

The present invention is characterized in that it is intended to use the hardened sclerotia themselves in biological control of without using mycelial culture or suspension.

In order to solve the problem of conventional technology related to microbial products by focusing on the specialized structure of sclerotium-forming fungi, the sclerotium, the invention seeks to provide a composition that overcomes the problems in stages of production, storage, distribution and application, and exerts stable biological efficacy.

In addition, a method for effectively suppressing the growth of target plants like Bur Cucumbers using the composition of the present invention is to be provided.

Also, this invention was completed to provide a method to suppress the occurrence of soil-borne diseases in grasses like the dollar spot.

### Technical Solution

In order to solve the above problems, the present invention provides a solid composition containing dormant fungal sclerotia; and carbon source and nitrogen source nutrients, wherein the composition is dried.

In addition, the solid composition can be provided as containing one or more absorbent, binding, and preservative substances, the dormant sclerotium of the fungus is provided as one of the genus Sclerotinia, genus Sclerotium and genus Rhizoctonia, the dormant sclerotium of the fungus can be provided as one to five, either in spherical (globular) or extruded pellet formulations, and the composition can be provided as having 1 to 20wt% of the carbon source and nitrogen source nutrients.

In addition, the solid composition can be used for weed management or suppression of soil-borne diseases; weed management is used for weeding Bur Cucumber, and supression of soil-borne diseases can be one among dollar spot disease, large patch, or rice sheath blight.

### Effect of Invention

The present invention has the effect of providing a biological dormant sclerotium itself to the weed/disease management technology.

The present invention provides convenience of use in the commercialization process of the product such as in product production, storage, distribution, etc., convenience of use is also provided for storage and distribution in the field all by using sclerotium, and after application, the sclerotium can survive so that it is activated only if its environmental conditions are suitable.

The present invention by including the fungal nutrients in the solid composition containing the sclerotium, the carbon source necessary for the growth of the mycelium after germination, a nitrogen source, etc. it is possible to effectively supply the nutrient components to maximize the weed/disease control effect.

The present invention can provide an effect to further extend the moisture supply time required for germination of the sclerotium and mycelium growth during hydration such as rainfall in a natural state by supporting the inclusion of absorbent material.

The present invention comprises sclerotium of fungi, nutrients and absorbent materials together and further includes preservatives for inhibiting contaminant microorganisms so that there is an effect of inhibiting the growth of contaminant microorganisms competing for nutrients with sclerotium-forming fungi during the early hydration process where the sclerotium germinates and the mycelium grows.

The present invention can provide a method of management of a target plant with known pathogenicity to sclerotium-forming fungi such as the Bur Cucumber.

The present invention can provide an effective management method that can suppress the occurrence of soil-borne disease, such as the dollar spot disease, which occurs in the grass.

The present invention can provide convenient use and economic efficiency to product producers, distributors, and field users.

In addition, the effect of the present invention is not limited to those mentioned above, other effects not mentioned will have to be recognized from the following specific descriptions.

### [BRIEF DESCRIPTION OF THE FIGURES]

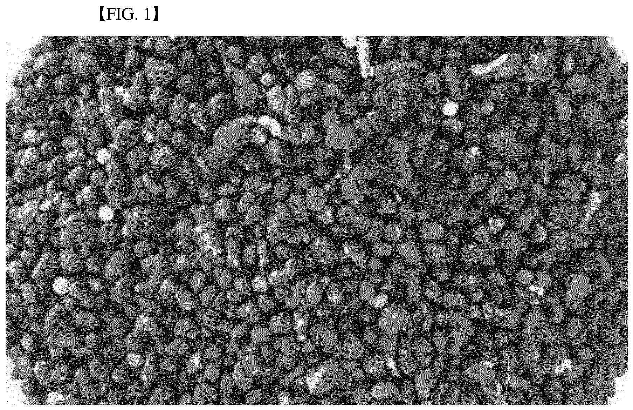

FIG. 1 is a photograph of a sclerotium used in the sclerotium composition experiment of the present invention.

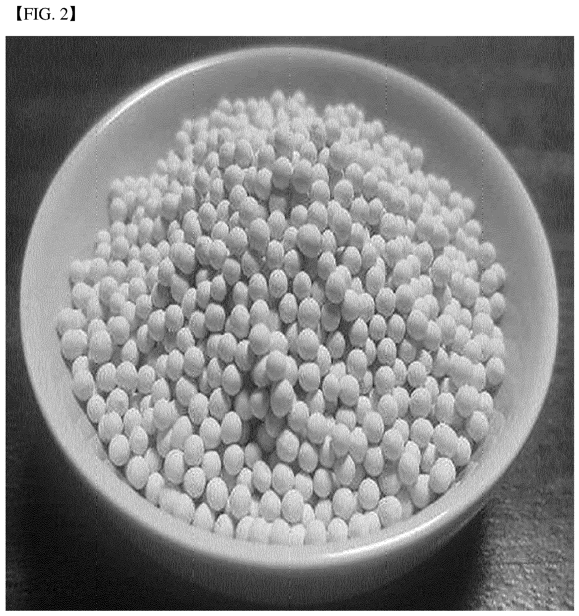

FIG. 2 is a photograph of a spherical pellet preparation containing the sclerotium.

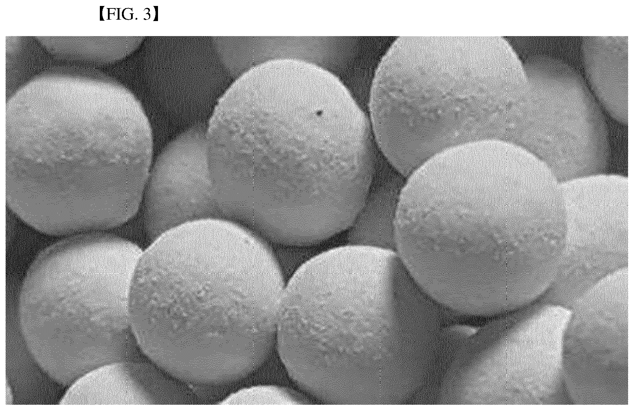

FIG. 3 is an enlarged photograph of a spherical pellet preparation.

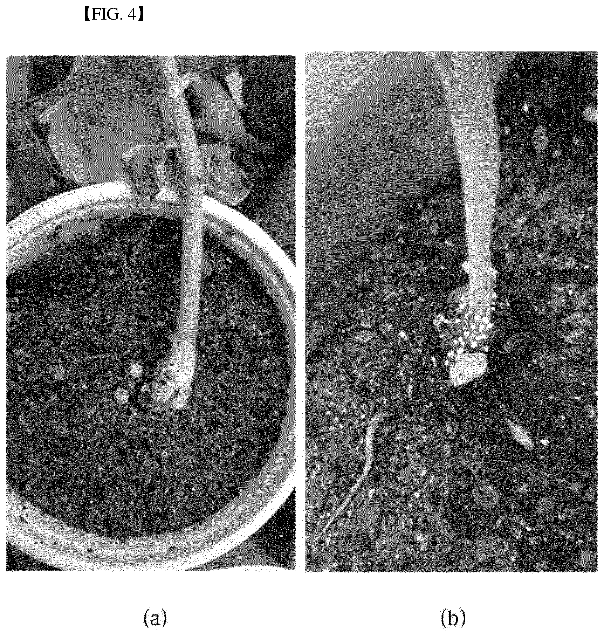

FIG. 4 is a pathogenic investigation photograph of the Bur Cucumber seedlings by the *Sclerotinia trifoliorum* BWC98-105 strain conducted in the present invention, the mycelium grows in the underground portion of the seedling, showing the symptoms of damping-off in the underground portion of the seedling, it can be confirmed that the sclerotium is formed.
FIG. 5 is a photograph showing the foliage coverage(B) significantly reduced because of the withering of the Bur Cucumber compared to the foliage coverage(A) of the untreated control group in the area sprayed with a spherical pellet preparation of *Sclerotinia trifoliorum* BWC98-105 strain in the natural environment.
FIG. 6 is a photograph of the sclerotium re-formed on and at the stem of the Bur Cucumber infected and withered by the BWC98-105 strain after spraying a spherical pellet preparation of the *Sclerotinia trifoliorum* BWC98-105 strain in the natural environment.

### Best Mode for the Invention

In the description of the present invention, a specific description of the related known function or configuration is omitted if it is determined that it may blur the main point of the present invention.

Also, the terms described later are terms defined in consideration of the function in the present invention, it may vary depending on the user, the intention of the operator or the precedent. Therefore, the definition should be based on the contents throughout the present description as a whole.

The present invention relates to sclerotium composition and its use to provide a biological dormant sclerotium itself to the weed/disease control technology.

The present invention provides a solid composition for plant management, characterized in that it contains the sclerotium of fungi, the solid composition may further contain nutrients necessary for the growth of sclerotium-forming fungi, or the solid composition may further include an absorbent material.

Fungi of the present invention may be any one of the genus Sclerotinia, genus Sclerotium and genus Rhizoctonia, nutrients include sugar or glucose as a carbon source, nitrogen source includes soybean meal or yeast extract, the total content of nutrients containing a carbon source and nitrogen source may be 1 to 20wt% in the solid composition.

In the present invention, the absorbent material may be a highly absorbent polymer that can absorb water more than 10 times the weight of the material itself.

The solid composition of the present invention can be provided for weed or soil-borne disease management; the weed management may be for Bur Cucumber or white clover, and the soil-borne disease management may be for dollar spot disease, grass large patch, or rice sheath blight.

The solid composition of the present invention includes one to five sclerotia, in the form of either globular or extruded pellet.

The sclerotium of the fungus of the present invention can provide a method for weeding the target plant, or it can provide a method for using the sclerotium of the fungus for soil-borne disease management.

The sclerotium composition of the present invention provides a bio-herbicide function to selectively remove target plants such as Bur Cucumber and provides a bio-fungicide function to effectively suppress soil-borne disease such as dollar spot disease of the grass. However, it is not limited to this meaning and can be defined as a term that can contain all conceptually equivalent levels of components.

Soil-borne diseases can be defined as a disease in which a pathogen infringes on the main crop when it was dormant or proliferating and then latent in the soil before.

The target plant is a plant subject to management and removal such as the Bur Cucumber and white clover.

### Implementation Form of Invention

### Preparation

Fungi used in the present invention can be one of the fungi reported to form sclerotium (Smith ME, Henkel TW., Rollins JA. 2015. How many fungi make scleortia? Fungal Ecology 13, 211-220) and among the fungi reported it can be made up of the sclerotium of the plant pathogenic fungi.

Among the sclerotium forming fungi, in particular, it may be sclerotium obtained from fungi in one or more of the genus Sclerotinia, Sclerotium, or Rhizoctonia, the sclerotium composition contains nutrients including carbon sources, nitrogen sources, etc. or trace elements required for germination and growth of the sclerotium, but it can be designed to vary depending on the target plant.

The absorbent material used in the sclerotium composition preferably has the property of absorbing more than 10 times water of its own weight, thereby providing the effect of extending the moisture supply time so that nutrients and water can promote germination and growth of sclerotium.

The sclerotium composition of the present invention may include one or more extenders, preservatives, or adhesives as supplementary materials.

Hereinafter, the present invention will be described in more detail through examples.

### Example 1. Comparison of viability of sclerotium in a dry environment

The viability of the sclerotium was compared with the mycelium culture in a dry environment. As for the sclerotium-forming fungi, sclerotium (hereinafter referred to as "sclerotium 1") of the *Sclerotinia trifoliorum* BWC 98-105 (deposit number: KACC93004P, hereinafter referred to as "sclerotium-forming fungus 1") and sclerotium (hereinafter referred to as "sclerotium 2") of the *Sclerotium delphinii* BWC04-107 (deposit number: KACC93031P, hereinafter referred to as "sclerotium-forming fungus 2") are used and sclerotium of the fungi mentioned here was compared with mycelium culture of the mycelium culture state and the cured sclerotium itself.

The sclerotium of sclerotium-forming fungi was obtained by incubation at 25°C for more than 2 weeks in potato dextrose agar (PDA; Difco Inc.), mycelium culture of sclerotium-forming fungi was obtained from being cultured at 25°C for 5 days in the PDA to secure the product in the stage before sclerotium formation.

The mycelium culture is obtained by cutting the section of the PDA where the sclerotium-forming fungi are growing (horizontal 1 cm x vertical 1 cm x thickness 0,5 cm) without contamination, but two sets each are prepared for both sclerotium and mycelium culture, one set is cultured at 25°C after being treated in potato dextrose agar without the drying process, the other set is cultured in potato dextrose agar at 25°C after being dried at 50°C for 3 days in a dryer (dry oven).

The experiment was repeated 5 times and compared the viability by measuring the diameter of the fungus colony growing in potato dextrose agar.

**[Table 1]**

| Viability of Sclerotium-forming Fungi After Drying Process | | | | | | |
|---|---|---|---|---|---|---|
| Fungi | Diameter of Mycelial Growth on Potato Dextrose Agar (cm) | | | | | |
| | Cultured Without Drying Process | | | Cultured After Drying(50°C, 3 days) | | |
| | 1 Day(s) | 3 Day(s) | 7 Day(s) | 1 Day(s) | 3 Day(s) | 7 Day(s) |
| Mycelium Culture of Sclerotium-forming Fungus 1 | 1.0 | 3.2 | 5.0 | 0 | 0 | 0 |
| Mycelium Culture of Sclerotium-forming Fungus 2 | 0.8 | 3.0 | 7.5 | 0 | 0 | 0 |
| Sclerotium 1 | 0 | 2.5 | 7.5 | 0 | 2.2 | 6.5 |
| Sclerotium 2 | 0 | 2.2 | 7.2 | 0 | 2.0 | 6.1 |

As shown in [Table 1], the sclerotium of the sclerotium-forming fungi of the present invention survive in a drying environment for 3 days at 50°C, confirming that it will retain its vitality in the poor conditions of the production, storage, and the distribution stages, but the already active mycelium cultures are all inactivated in the drying process with the mycelium not growing even after a certain time. Therefore, the composition of the present invention was confirmed that it can effectively survive in a dry environment frequently exposed to in a real implementation environment by confirming the characteristics of the sclerotium surviving in a dry environment.

### Preparation Example 1 to Preparation Example 7. Preparation of the sclerotium composition

Based on Example 1, the solid composition was completed in the same composition ratio as in [Table 2]. First, the prepared sclerotium is placed in the tablet coating machine and the rest of the materials of the composition in powder form is added while the machine is rotating, and then the water is properly sprayed while being coated and the process of adding powder and spraying of water is repeated again and again to make a spherical pellet about 4 mm in diameter and the pellet is dried to complete the composition of the Preparation Example. [Table 2] Pellet composition ratio is the composition ratio that excludes moisture.

As a carbon source in the composition, commercially available glucose powder was used, soybean meal powder was used as a nitrogen source, and the citric acid powder was used as a preservative. Sodium alginate powder was used as an absorbent material and adhesive during pellet formulation, and the extender used commercially available silica powder on the market. The total content of nutrients, including carbon sources or nitrogen sources, may be appropriately adjusted in the solid composition according to the needs of the customer within 1 to 20wt%.

**[Table 2]**

| Constitution of Composition | | | | | | | |
|---|---|---|---|---|---|---|---|
| Composition | Constitution of Composition(Unit: Part by Weight) | | | | | | |
| | Sclerotium 1 | Sclertoium 2 | Glucose | Soybean meal | Citric Acid | Sodium Alginate | Silica |
| Preparation Example 1 | 2 | 0 | 10 | 1 | 2 | 5 | 80 |
| Preparation Example 2 | 0 | 2 | 10 | 1 | 2 | 5 | 80 |
| Preparation Example 3 | 2 | 0 | 0 | 0 | 0 | 5 | 93 |
| Preparation Example 4 | 0 | 2 | 0 | 0 | 0 | 5 | 93 |

In addition, in order to contrast with these Preparation Examples, Comparative Examples 1 and 2 were prepared using a mycelium culture before the sclerotium of the two experimental strains is formed as a control group, Comparative Example 1 is a mycelium culture of *Sclerotinia trifoliorum* BWC98-105, Comparative Example 2 is a mycelium culture of *Sclerotium delphinii* BWC04-107.

### Example 2. Weeding effect on Bur Cucumber in greenhouse conditions

Using the Preparation Example 1-4 and Comparative Example 1, the weeding effect was confirmed through the pathogenic test for the Bur Cucumber seedlings.

After germinating the Bur Cucumber seeds, they were sown in the upper soil of each pot (diameter 8.5 cm, height 6.5 cm) and bred in the greenhouse, in the growth stage where there are 2-3 main leaves in the seedling and each Preparation Example and Comparative Example composition were treated around the Bur Cucumber vine. The five pieces of spherical pellet of each Preparation Example were used for each Bur Cucumber seedlings, in each Comparative Example the mycelium culture was used by cutting a section (1 cm wide x 1 cm long x 0.5 cm thick) of the potato dextrose agar in which the seclortium-forming fungi grow.

On the other hand, the Preparation Example and the Comparison Example were designed with 10 Bur Cucumber seedlings for each example for a total of two sets. One set of was always supplied with sufficient moisture by bottom watering after the treatment of Preparation Examples 1 and 2, and the other set was sprayed with a minimum amount of water every day for a week while being managed at a level that the Bur Cucumber does not wilt and from the 8th day the bottom watering was used to ensure that sufficient moisture was supplied, the pathogenicity of each example was studied, and the results are shown in Table 3 below.

**[Table 3]**

| Pathogencity Towards Burcucumber Seedlings | | | | | | |
|---|---|---|---|---|---|---|
| Composition | Pathogencity Towards Burcucumber Seedlings | | | | | |
| | Moisture Continuously Supplied to Soil Using Bottom Watering System | | | Moisture Supplied to Soil Using Bottom Watering System After 1 Week of Dry Conditions | | |
| | 7 Days | 14 Days | 21 Days | 7 Days | 14 Days | 21 Days |
| Preparation Example 1 | + | ++ | +++ | - | + | ++ |
| Preparation Example 2 | + | ++ | +++ | - | + | ++ |
| Preparation Example 3 | - | + | + | - | - | + |
| Preparation Example 4 | - | + | + | - | - | + |
| Comparative Example 1 | ++ | +++ | +++ | - | - | - |
| Comparative Example 2 | ++ | +++ | +++ | - | - | - |

In the display of [Table 3], '-' is that all 10 objects are not pathogenic, '+' is that mycelium is observed in less than 3 out of 10 objects, but there are no wilting symptoms, '++' is that mycelium is observed in more than 5 out of 10 objects and weak wilting symptoms appear, '+++' is that mycelium is observed in more than 9 out of 10 objects and wilting is to such an extent that the plant turn brown from drying and the plant is withering away.

As a result of the experiment, as in [Table 3] Preparation Example 1 and 2 showed a stable pathogenicity for the Bur Cucumber seedlings, although the pathogenicity can be confirmed in Preparation Example 3 and 4, the expression is late and the effect was low. That is, Comparative Examples 1 and 2 both showed rapid pathogenic expression in bottom watering conditions, but the pathogenicity did not appear in the conditions that limited the water supply without bottom watering for a week. Through this, Preparation Examples 1 and 2 confirmed that even in inconsistent conditions with both dry and humid conditions as in the natural environment stable pathogenicity is expressed. Also, it can be confirmed that the active mycelium culture of Comparative Examples 1 and 2 are inactivated if the moisture supply from the time of use is not enough, so it was confirmed that there is a significant limit to the commercial introduction of the formulation.

### Example 3. Weeding effect on Bur Cucumbers in the natural environment

Experiments in the natural environment were conducted near Seoul from early April 2018, when the natural occurrence of Bur Cucumbers was observed. First, the Bur Cucumber colony was divided into test plots (size 4 m x 5 m), and the Preparation Example 1 and 2 of Table 2 were sprayed evenly at a level of 10 g per square meter (=200 g/test strip) three times (1st May 20, 2nd June 30, 3rd July 15). Furthermore, one test plot of the same size was used as an untreated control group. At the time of the 1st spraying, the Bur Cucumber stems began to creep over the surface, exceeding 1 m in length. The average temperature, rainfall day, and rainfall for 35 days after the 1st spraying were 21.3°C, 12 days, and 206.5 mm, respectively, the average temperature, rainfall day, and rainfall for 35 days after the 2nd spraying were 26.9°C, 14 days, and 305.6 mm, respectively, and the average temperature, rainfall day, and rainfall for 28 days after the 3rd spraying were 30.0°C, 9 days, and 35.0 mm, the conditions in the three months of trial being quite irregular in terms of temperature and moisture supply.

The germination of the Bur Cucumber was observed in early April, the average temperature of the 1st spraying day, May 13, was 15 °C, the minimum temperature was 12.7 °C, and the maximum temperature was 20.0 °C. At this point, the Bur Cucumber had 5-10 main leaves. The weeding effect on the Bur Cucumber of Preparation Example 1 and 2 was evaluated as foliage coverage, the foliage coverage is the surface area covered by the Bur Cucumber in the test area determined by the naked eye with 100% being based on the coverage of the untreated control group, and the foliage coverage was calculated at each spraying and the results of a longitudinal survey confirmed the effects as shown in Table 4.

**[Table 4]**

| Foliage Coverage Survey of Natural Colony of Burcucumber Vines | | | |
|---|---|---|---|
| Composition | Foliage Coverage of Burcucumber Vines(%) | | |
| | 1st Survey | 2nd Survey | 3rd Survey |
| | June 24th | July 29th | August 26th |
| | 35 Days After 1st Spraying | 70 Days After 1st Spraying | 98 Days After 1st Spraying |
| | | 35 Days After 2nd Spraying | 63 Days After 2nd Spraying |
| | | | 28 Days After 3rd Spraying |
| Preparation Example 1 | 100 | 20 | 15 |
| Preparation Example 2 | 100 | 30 | 20 |
| Untreated Control Group | 100 | 100 | 100 |

As can be seen in [Table 4], the foliage coverage of Preparation Examples 1 and 2 at the time of the 2nd survey was reduced to levels of 20-30%, and that was maintained by the time of the 3rd survey. Through this, Preparation Examples 1 and 2 confirmed that even in the natural environment with inconsistent temperature, humidity, etc. weeding effect on Bur Cucumber was highly effective. That is, although it was not possible to observe the remedial effects at the time of the 1st survey, at the time of the 2nd survey the foliage coverage of the test plot treated with Preparation Examples 1 and 2 of the present invention was significantly lower than the non-treated control group that did not take any action in the test plot, the reason the difference in the foliage coverage was not observed at the time of 1st survey is that the Bur Cucumber vines grew to a length of 1-3 m but it did not grow enough to cover the surface, another reason is that the average temperature was low at 21.3°C and there was low humidity as it was before the start of the rainy season.

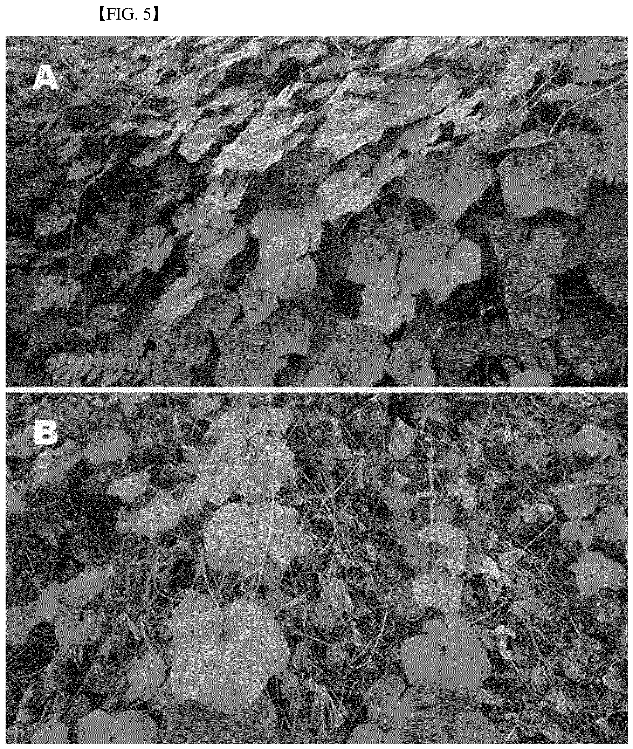

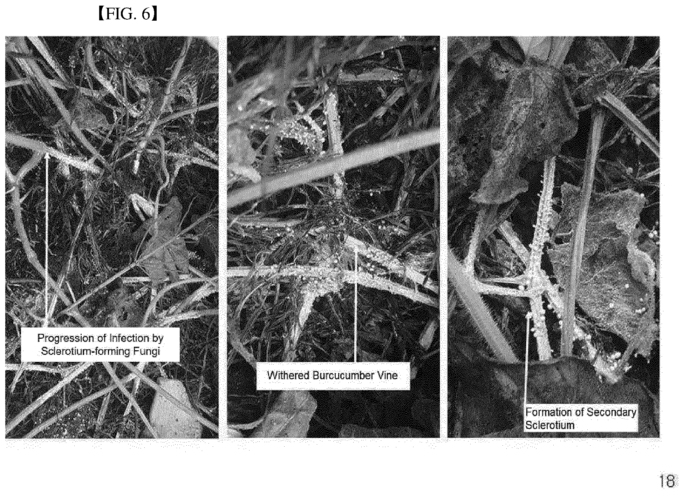

However, at the time of the 2nd survey there having been an additional 2nd spraying as well as the rise in temperature and humidity, the foliage coverage of the Bur Cucumber in the test plot treated with Preparation Example 1 and 2 was significantly lower than the untreated control group, as shown in the [Figure 5] photo, the foliage coverage of Bur Cucumber at the time of the 2nd spraying was confirmed as being significantly different. Also, a closer look at the region with low foliage coverage in the [Figure 6] photo shows that as the mycelium of the sclerotium-forming fungi in the Bur Cucumber grows and infects the target plant it could be observed that there were blight symptoms in the vines and secondary sclerotium being formed again. This supports the notion that sustainable management of the local target plant will be possible if the sclerotium is not lost. However, in the untreated control group as shown in the [Figure 5] photo, the blight symptoms in the vines or sclerotium formation could not be observed.

In addition, after spraying the composition of the present invention, in the test plot of Preparation Examples 1 and 2 at the time of the 3rd survey, the foliage coverage of Bur Cucumber was maintained at an even lower level. Through this, even in the natural environment where the change in temperature and humidity is large and there is direct sunlight, the composition of Preparation Example 1 and 2 survives despite it not being active, after the rainy season, in high temperature and humid environmental conditions, it is activated and pathogenicity to the Bur Cucumber is expressed and the growth suppression effect was confirmed to be excellent. Also, in the empty space due to the low foliage cover, non-target plants such as goosefoot, *Solanum nigrum,* and green foxtail, etc. were settled intact.

### Example 4. Weeding effect on white clover

Example 4 consists of spraying Preparation Example 1 and 2 on the soil to check the weeding effect on white clover. The experiment was conducted in a glass greenhouse, where a colony of white clovers that grows naturally were transferred to a square pot (45 cm wide x 30 cm long x 5.5 cm high), planted and raised in a greenhouse, and was managed for three weeks to ensure that the white clovers settled and grew enough.

After spraying the Preparation Example 1 and 2 evenly at a level of 10 g per square meter, five pots were supplied with enough moisture with bottom watering and there were five pots that did not use bottom watering managed in as dry soil conditions as possible without the white clovers wilting. The weeding effect was evaluated by calculating the area ratio (%) of white clover that did not grow compared to the area of the square pot, and the result of the investigation are shown in [Table 5].

**[Table 5]**

| Survey of the Weeding Effect on White Clover | | | | | | |
|---|---|---|---|---|---|---|
| Composition | Average Weeding Effect on White Clover(%)' | | | | | |
| | Moisture Continuously Supplied to Soil Using Bottom Watering System | | | Moisture Supplied to Soil Using Bottom Watering System After 1 Week of Dry Conditions | | |
| | 14 Days after application | 21 Days after application | 28 Days after application | 14 Days after application | 21 Days after application | 28 Days after application |
| Preparation Example 1 | 10.0 ± 5 | 30.0 ± 5 | 80.0 ± 16 | 0 | 20.0 ± 9 | 68.3 ± 10 |
| Preparation Example 2 | 0 | 23.3 ± 6 | 73.3 ± 13 | 0 | 10.0 ± 9 | 61.7 ± 13 |
| Preparation Example 3 | 0 | 6.7 ± 3 | 26.7 ± 5 | 0 | 0 | 5.0 ± 5 |
| Preparation Example 4 | 0 | 5.0 ± 5 | 20.0 ± 5 | 0 | 0 | 5.0 ± 0 |
| Comparative Example 1 | 30.0 ± 13 | 66.7 ± 12 | 85.0 ± 13 | 0 | 0 | 0 |
| Comparative Example 2 | 15.0 ± 10 | 50.0 ± 10 | 71.7 ± 10 | 0 | 0 | 0 |

In [Table 5] the Preparation Examples 1 and 2 were confirmed to express pathogenicity for white clover and it was possible to check that pathogenicity existed in Preparation Examples 3 and 4, but it was expressed later than in Preparation Examples 1 and 2 and the level was also in a very low state.

However, in Comparative Examples 1 and 2, the pathogenicity was expressed quickly and in high levels with bottom watering, but in the dry conditions with no bottom watering for one week after spraying, almost no weeding effect was expressed, showing that the examples became inactive. That is, although Preparation Examples 1 and 2 took some time to reach its initial activation stage, high levels of pathogenicity were expressed and the weeding effect was maintained at high levels for a long time, Comparative Examples 1 and 2 was in the active state from the beginning and initially expressed high pathogenicity, but in a dry environment it rapidly became inactivated and there were no subsequent weeding effect making it difficult to be used in any real life applications.

In the end, it was confirmed that the sclerotium composition of Preparation Examples 1 and 2, unlike the mycelium culture, was able to effectively control the white clover over a long period of time even in a natural state.

### Example 5. Investigation of the growth suppression effect for Sclerotinia dollar spot

Grass dollar spot disease-causing pathogen *Sclerotinia homoeocarpa* is taxonomically similar from a biological point of view to the sclerotium-forming fungus *Sclerotinia trifoliorum* used in the present invention sharing the same genus name, but *Sclerotinia trifoliorum* BWC98-105 used in the composition of the present invention differs in that it is reported as having no pathogenicity to the grass. Also, the sclerotium-forming fungus *Sclerotium delphinii* is taxonomically different, but ecologically similar in that it is a soil-borne pathogenic fungus. Because of this, the composition Preparation Example 1 and 2 of the present invention can preempt the space where the grass grows when sprayed prior to the occurrence of the dollar spot disease and when the composition's fungi are settled in, this means that the ecological competition of the composition's fungi with the dollar spot disease fungi will reduce the occurrence of the disease. This was confirmed through Example 5.

Example 5 was carried out in a glass greenhouse using the Preparation Examples 1 and 2. It evaluated whether the inhibitory effect on the dollar spot disease, Sclerotinia homoeocarpa exists. In other words, Sclerotinia dollar spot disease is pathogenic to most grass types regardless of its type, so creeping bentgrass (*Agrostis palustris*), a kind of western grass was used, the square pot(width 45 cm x length 30 cm x height 5.5 cm) was properly filled with plant-growing top soil, and then the creeping bentgrass carpet cut to fit the square pot was planted, then it was grown in the greenhouse for 3 weeks. And the Preparation Example 1 and 2 was sprayed at a level of about 10 g per square meter (=1.5 g / pot) and it was grown for one week while being bottom watered, and then five holes(diameter 1 cm, depth about 2 cm) were dug on the surface of the surface where grass was growing and each hole was inoculated with 3 g of soil inoculum of the dollar spot disease fungus. The soil inoculation of the dollar spot disease fungus was made by mixing components in a weight ratio of oatmeal:sand:water=1:20:40, and then putting in mycelium culture grown in potato dextrose agar and culturing it at 25°C for 10 days. In the analysis of the experimental results, the inhibitory effect on the dollar spot disease was evaluated using the damaged area ratio of bentgrass growing in the square port, that is, the damage area caused by the occurrence of the dollar spot disease was divided by the area of the square port area then the figure was multiplied by 100, the results can be seen in [Table 6].

**[Table 6]**

| Supression Effect on Grass Dollar Spot Disease | | | |
|---|---|---|---|
| Composition | Grass Dollar Spot Disease Damage Area Ratio(%)¹ | | |
| | 14 Days After Spraying | 21 Days After Spraying | 28 Days After Spraying |
| Preparation Example 1 | 2.0 ± 1 | 5.0 ± 2 | 8.0 ± 6 |
| Preparation Example 2 | 2.0 ± 1 | 5.3 ± 1 | 9.3 ± 5 |
| Untreated Control Group | 5.0 ± 3 | 25.0 ± 8 | 47.0 ± 15 |

That is, in [Table 6] the case of Preparation Examples 1 and 2 of Example 5 showed that the degree of damage after spraying was confirmed to be at about 8% after 28 days, but in the untreated control group the initial damage was similar, but after 28 days it soared to about 47%. This shows that there is a significant inhibitory effect on the dollar spot disease afflicting creeping bentgrass in the case of Preparation Examples 1 and 2, and at the same time it confirms that there is no pathogenicity for the grass and creeping bentgrass, which are not target plants but plants to be managed and protected.

In the end, Example 5 showed that, even if the Preparation Example 1 and 2 are used in a state where the environmental conditions are irregular, the dollar spot disease of the grass is very effectively inhibited, but the grass is not harmed, demonstrating a positive effect. Through this, although not presented as an Example in the present invention, by applying the point that there is a pathogen inhibitory effect through ecological competition, grass large patch (pathogen *Rhizoctonia solani*) and rice sheath blight (pathogen *Rhizoctonia solani*) can be expected to have a similar effect when using the sclerotium composition. These effects, for a person skilled in the art, it will be possible to carry out various modifications and changes to the present invention within a scope that does not deviate from the idea and area of the present invention described below in the scope of the claims in the patent.

Also, the application amount per area and the number of sprays for the composition of Preparation Examples 1 and 2 were not evaluated separately but it can be reasonably assumed that the length of efficacy will scale proportionately to application amount per area and the number of sprays. However, since the activation of the sclerotium must occur for weeding and pathogen suppression effects, the environmental conditions such as temperature, humidity, or direct sunlight must be taken account in deciding the application amount and the number of sprays in the commercialization stage.

As described above, the composition Preparation Examples 1 and 2 of the present invention was confirmed as having the effect of effectively suppressing the Bur Cucumber, a ecologically harmful species, in greenhouse and natural environmental conditions. In addition, it was clearly confirmed as being highly effective in suppressing the target plant white clover and also very effective in suppressing the Sclerotinia dollar spot disease.

In the present invention it has been described with reference to the above Examples for experimental convenience, but for a skilled person in the art, it will be possible to carry out various modifications and changes to the present invention within a scope that does not deviate from the idea and area of the present invention described below in the scope of the claims in the patent.

### Industrial Applicability

The present invention can be used industrially for plant management and weeding.

## Claims

1. A solid composition containing dormant sclerotia comprising
fungal dormant sclerotium; and
carbon source and nitrogen source nutrients,
wherein the composition is dried.

2. The solid composition of claim 1, wherein the solid composition further comprises any one or more of absorbent materials, adhesives, or preservatives.

3. The solid composition of claim 1, wherein the fungal dormant sclerotium is one of genus Sclerotinia, genus Sclerotium, and genus Rhizoctonia.

4. The solid composition of claim 1, wherein the fungal dormant sclerotium includes one to five sclerotia.

5. The solid composition of claim 1, wherein the form of the solid composition is either a globular or an extruded pellet.

6. The solid composition of claim 1, wherein the carbon source and nitrogen source nutrients constitute 1 to 20wt% of the solid composition.

7. A method of using the solid composition containing dormant sclerotium of any one of the claims 1 to 6 for weed management.

8. A method of using the solid composition containing dormant sclerotium of any one of the claims 1 to 6 for suppressing fungal soil-borne diseases.

9. The method of claim 7, wherein the use of solid composition for weed management containing dormant sclerotium is for controlling Bur Cucumber.

10. The method of claim 8, wherein the fungal soil-borne disease is one of Sclerotinia dollar spot disease, grass large patch, or rice sheath blight.
